(19) **Europäisches Patentamt**

**European Patent Office**

**Office européen des brevets**

(11) **EP 0 630 217 B1**

(12) **EUROPEAN PATENT SPECIFICATION**

(45) Date of publication and mention
of the grant of the patent:
**10.05.2000 Bulletin 2000/19**

(51) Int. Cl.7: **A61F 2/24**

(86) International application number:
**PCT/SE93/00219**

(21) Application number: **93906940.7**

(22) Date of filing: **11.03.1993**

(87) International publication number:
**WO 93/17637 (16.09.1993 Gazette 1993/22)**

(54) **CARDIAC VALVE**

HERZKLAPPE

VALVE CARDIAQUE

(84) Designated Contracting States:
**AT BE CH DE DK ES FR GB GR IE IT LI LU MC NL
PT SE**

(30) Priority: **13.03.1992 SE 9200779**

(43) Date of publication of application:
**28.12.1994 Bulletin 1994/52**

(73) Proprietor: **JCL TECHNIC AB
S-104 25 Stockholm (SE)**

(72) Inventor: **LENTELL, Jan
S-112 43 Stockholm (SE)**

(74) Representative:
**Burman, Tore et al
AWAPATENT AB,
Box 45086
104 30 Stockholm (SE)**

(56) References cited:
**EP-A- 0 383 676        US-A- 3 938 197
US-A- 4 820 299        US-A- 5 078 739**

EP 0 630 217 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Xerox (UK) Business Services
2.16.7 (HRS)/3.6

## Description

### BACKGROUND OF THE INVENTION

### Field of the invention

The present invention relates to a back valve, more particularly a cardiac valve, so called heart valve, intended to allow flow of blood in one direction in a path of blood in connection to the heart of a living mammal, including man.

### Prior Art

Cardiac valves, so called heart valves, are found in a number of embodiments made both of wholly artificial materials, such as titanium, pyrolytic carbon or the like, or manufactured from natural tissue of porcine or bovine origin, said materials being treated in a chemical manner so as to obtain desirable properties. All hitherto known back valves for such intended function are associated with severe disadvantages, among which the following may be mentioned.

The designs not based on natural tissue are all made as more or less complicated constructions which result in risk of failure or unsatisfactory function in other ways. Known valves are, furthermore, designed so as to occupy, in an open position, an essential part of the flow cross-section, thus offering resistance to the flow of blood. Many of the known valves are furthermore designed in an asymmetric way with concomitant inconveniences. Furthermore, the prior art cardiac valves have a design where protruding parts disturb the flow of blood and also result in undesired epithelial growth. Finally, it can be mentioned that known valves often are associated with inertia in their opening or closing movement which substantially reduces their efficiency.

Examples of prior art designs are found in US patents Nos. 4,820,299 and 5,078,739. These known cardiac valves are subject to the disadvantages indicated above.

### SUMMARY AND OBJECTS OF THE INVENTION

The present invention has for a purpose to provide a cardiac valve, wherein the disadvantages of the prior art are eliminated or at any rate essentially reduced.

Another object of the invention is to provide a cardiac valve which in an open position offers low resistance to the flow of blood in view of the fact that the opening ratio, i.e. the ratio between the open valve passage and the cross-section in association with the valve position, is high.

Yet another object of the invention is to provide a valve showing a symmetric opening pattern, so that the flow of blood will not be disturbed in a non-desirable manner.

Still another object of the invention is to provide a cardiac valve having the capability of rapid opening and closing resulting in improved efficiency.

Another object of the invention is to provide a cardiac valve having a minimum of protruding parts whereby undesired epithelial growth is prevented or at least radically reduced.

For these and other objects which will be clear from the following description the cardiac valve according to the invention comprises a sleeve or a ring having an inlet end and an outlet end and which is provided with a central opening, and openable and closable flaps having for their purpose to allow flow of the blood through the central opening of the ring in the one direction but to prevent flow of the blood in an opposite direction. The flaps are designed as circle segments, the sector angle designated $\alpha$ having the value $\frac{360°}{n}$, where n is the number of circle segments. Said flaps are at their outer edge pivotably connected to the inside of the ring at said inlet end thereof, whereby said flaps in a closed position cooperate to form a sealing body and in an open position allow a free passage of blood.

The number of circle segments is at least three and at most six, and it is particularly preferred that the number of flaps is three so that the design shall be as simple as possible.

The invention is characterized in that the designed as curved elements so that they together in a closed position form a cone having an obtuse peak angle designated $\beta$. For practical reasons this peak angle is at most about 180° and suitably lies within the range about 150-175°. In this manner sealing cooperation between the flaps will be obtained in a closed position.

In accordance with the present invention each flap is connected to the inside of the ring by means of a hinge which is arranged in the center of the outer edge of the flap and which is accomodated in a recess in said ring. For the purpose of avoiding operational disturbances in the use of the cardiac valve it is suitable to design the attachment to the ring in such a manner that a certain flap alone cannot be folded to a position substantially exceeding the position, as seen in the closing direction, it has in cooperation with the other flaps.

In a manner known per se it is suitable to provide the ring at its axial ends with circumferential flanges, the function of which is to form a recess, wherein a suture ring can be placed for fixation to the surrounding tissue in connection with the implantation of the cardiac valve.

For design reasons it is preferred that the ring, at said one end where the flaps are pivotally attached, is provided with one circumferential flange which is directed inwardly substantially radially, against the inwardly facing side surface of which the outer edges of the flaps rest in sealing cooperation. In this manner also the preferred situation arises that a certain flap cannot alone be folded from an open position to a position substantially exceeding the position at which all the flaps rest in sealing cooperation.

Each hinge contains a pin or stud which is attached to the ring in a tangentially extending aperture therein.

In order to obtain efficient closing performance it is preferred that said flaps in a fully open position take an angle to a line parallel with a center line (L) to said ring which is greater than zero and smaller than about 7°.

With regard to the choice of material in the cardiac valve according to the invention any conventional material can be used. It is preferred to use titanium or pyrolytic carbon, optionally in combination, and the suture ring can in a usual manner be constituted by a biologically acceptable material, such as teflon, polyester, dacron, polyurethane, or the like.

Brief description of the drawing

The present invention will in the following be further described in relation to a preferred embodiment which is shown on the appended drawing. In the drawing:

> Fig. 1 shows a plan view on the cardiac valve according to the invention;
> Fig. 2 shows a side view, partly in section along A-A in Fig. 1; and
> Fig. 3 shows the three flaps separated and removed from the valve ring.

Description of a preferred embodiment

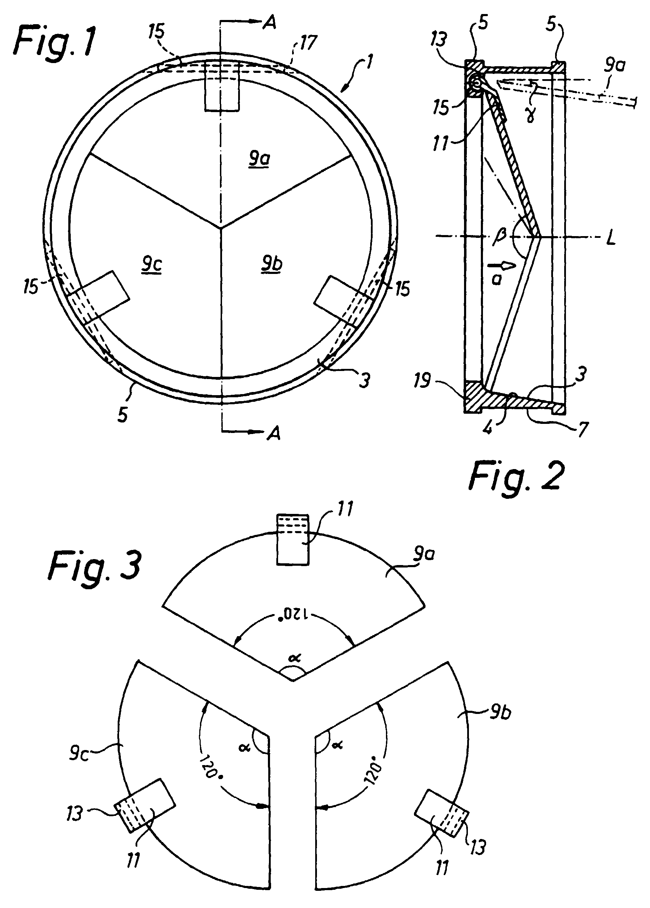

The cardiac valve generally designated 1 and shown in Figs. 1 and 2, consists in principle of a sleeve or ring 3 having a central passage 4, through which with the valve in an open position blood can pass the valve in the direction of arrow a) (Fig. 2). Furthermore, ring 3 is provided with two circumferential, outwardly extending flanges 5, one at each end of ring 3, said flanges 5 between themselves forming a recess 7 intended to accommodate the so called suture ring (not shown in the drawing).

Ring 3 accommodates three flaps 9a-c, each shaped as a circular segment as seen in the plane of the paper, where thus the sector angle $\alpha$ has a value 360:3, i.e. 120°. With their curved or arcuate form flaps 9a-c form a conical body having a peak angle $\beta$ (Fig. 2) which in the example shown is about 150°. This peak angle may of course be substantially larger but does suitably not exceed 180°.

Each flap 9 is at the outer edge of the circular segment provided with a hinge 11 having an enlarged outer end 13 to accommodate a hinge pin or stud 15 for providing the intended function. It is important to note that each hinge 11 is accomodated in a recess on the inside of the inlet side of ring 3. In this manner the presence of protruding parts will be avoided thereby preventing disturbing epithelial growth in the cardiac valve upon implantation. Pin or stud 15 is in the embodiment shown held in an aperture 17 extending tangentially through ring 3 enabling exterior assembly.

For practical reasons ring 3, at the end of the pivotable attachment of flaps 9a-c, provided with a radially inwardly directed circumferential flange 19, and the edge of flaps 9a-c rest in sealing cooperation against the inwardly directed side surface of said flange. This flange 19 has furthermore for its function to prevent that each separate flap in the closing direction can move past the position according to Fig. 2 determined by flaps 9a-c in sealing cooperation.

In Fig. 2 flap 9a is shown in open position by dotted lines. In order to obtain a maximum cross section for the flow of blood with the valve in an open position, while yet obtaining efficient closing of the valve, it is preferred that the angle $\gamma$ is greater than zero and smaller than about 7°. In the embodiment shown the angle $\gamma$ is about 5°. Said angle $\gamma$ refers to a line parallel to the center line L of ring 3.

The embodiment of the cardiac valve according to the present invention as described above has several important advantages as compared to the prior art. Thus, the symmetry in the arrangement of the flaps means that the flow pattern with the valve in an open position becomes symmetric so that non-desirable turbulence in connection with the flow of the blood will be prevented. Furthermore, the design means that the opening ratio of the valve is very high, for example exceeding 90%, in relation to the free cross-section of the blood vessel in question. In this manner a minimum hindrance to the flow of the blood will be obtained with the valve in an open position. Furthermore, the design of the valve is simple and reliable and malfunction can therefore be eliminated. Finally, the described design means that the valve operates with very short opening and closing times, which is essential for effective function.

It can be added that the cardiac valve according to the present invention can be used both in aorta and as a mitral valve or otherwise in blood paths where a back valve function is required.

The present invention is not restricted to the embodiment described above with regard to details of construction or otherwise. It can, of course, be modified within the frame of the definition found in the appended claims.

Claims

1. Cardiac valve, so called heart valve, comprising a sleeve or ring which is provided with a central opening (4), and openable and closeable flaps having for their purpose to allow flow of blood though the central opening (4) in one direction, wherein the flaps (9a-c) are designed as circle segments, where the

sector angle ($\alpha$) has the value 360°/n, where n is the number of circle segments and is 3, 4, 5 or 6, said flaps (9a-c) being at their outer edge pivotally connected to the inside of the ring (3) at one end thereof, in that each flap (9a-c) is connected to the ring (3) by means of a hinge which is arranged in the center of the outer edge of the flap and which is accommodated in a recess in said ring (3), whereby said flaps in a closed position co-operate to form a sealing body and in an open position allow a free passage of blood, characterized in that the flaps (9a-c) are arcuate so as to form together in the closed position a cone having an obtuse peak angle ($\beta$) and in that each hinge contains a pin or stud (15) which is attached to the ring (3) in a tangentially extending aperture (17) therein.

2. Cardiac valve according to claim 1, characterized in that the number of flaps (9a-c) is 3.

3. Cardiac valve according to claim 1 or 2, characterized in that said peak angle ($\beta$) is at most 180°.

4. Cardiac valve according to claim 3, characterized in that said peak angle ($\beta$) lies within the range about 150 to 175°.

5. Cardiac valve according to any preceding claim, characterized in that the attachment to the ring (3) is shaped in such a manner that one flap (9a-c) alone cannot be folded to a position substantially exceeding the position it has in co-operation with the other flaps (9a-c).

6. Cardiac valve according to any preceding claim, characterized in that the ring (3) at the ends thereof is provided with circumferential flanges (5) forming a recess (7), wherein a suture ring can be placed for fixation to the surrounding tissue.

7. Cardiac valve according to any preceding claim, characterized in that the ring (3) at said one end thereof is provided with an inwardly directed circumferential flange (19), the outer edges of flaps (9a-c) sealingly engaging the inwardly facing side surface of said flange (19).

8. Cardiac valve according to any preceding claim, wherein said flaps (9a-c) in a fully open position take an angle ($\gamma$) to a line parallel with a center line (L) to said ring (3) which is greater than zero and smaller than about 7°.

9. Cardiac valve according to any preceding claim, characterized in that it is made of a material selected from titanium and pyrolytic carbon.

## Patentansprüche

1. Herzklappe, die eine Hülse bzw. einen Ring umfaßt, die bzw. der mit einer Mittelöffnung (4) sowie zu öffnenden und zu schließenden Klappen versehen ist, deren Zweck darin besteht, das Strömen von Blut durch die Mittelöffnung (4) in einer Richtung zu ermöglichen, wobei die Klappen (9a-c) als Kreissegmente ausgeführt sind und der Sektorwinkel ($\alpha$) den Wert 360°/n hat, wobei n die Anzahl der Kreissegmente ist und 3, 4, 5 oder 6 beträgt, die Klappen (9a-c) an ihrer Außenkante schwenkbar mit der Innenseite des Rings (3) an einem Ende desselben verbunden sind, wobei jede Klappe (9a-c) mit dem Ring (3) über ein Gelenk verbunden ist, das in der Mitte der Außenkante der Klappe angeordnet und in einer Aussparung in dem Ring (3) aufgenommen ist, so daß die Klappen in einer geschlossenen Position zusammenwirken und einen Dichtungskörper bilden und in einer geöffneten Position das ungehinderte Hindurchtreten von Blut ermöglichen, **dadurch gekennzeichnet**, daß die Klappen (9a-c) gebogen sind und in der geschlossenen Position zusammen einen Kegel mit einem stumpfen Scheitelwinkel ($\beta$) bilden, und dadurch, daß jedes Gelenk einen Stift bzw. Bolzen (15) enthält, der an dem Ring (3) in einer tangential verlaufenden Öffnung (17) angebracht ist.

2. Herzklappe nach Anspruch 1, **dadurch gekennzeichnet**, daß die Anzahl von Klappen (9a-c) 3 beträgt.

3. Herzklappe nach Anspruch 1 oder 2, **dadurch gekennzeichnet**, daß der Scheitelwinkel ($\beta$) höchstens 180° beträgt.

4. Herzklappe nach Anspruch 3, **dadurch gekennzeichnet**, daß der Scheitelwinkel ($\beta$) in dem Bereich von ungefähr 150 bis 175° liegt.

5. Herzklappe nach einem der vorangehenden Ansprüche, **dadurch gekennzeichnet**, daß die Anbringung an dem Ring (3) so geformt ist, daß eine Klappe (9a-c) allein nicht an eine Position geklappt werden kann, die im wesentlichen jenseits der Position liegt, die sie beim Zusammenwirken mit den anderen Klappen (9a-c) einnimmt.

6. Herzklappe nach einem der vorangehenden Ansprüche, **dadurch gekennzeichnet**, daß der Ring (3) an seinen Enden mit Umfangsflanschen (5) versehen ist, die eine Aussparung (7) bilden, in die ein Nahtring zum Befestigen an dem umgebenden Gewebe eingesetzt werden kann.

7. Herzklappe nach einem der vorangehenden Ansprüche, **dadurch gekennzeichnet**, daß der Ring (3) an dem einen Ende desselben mit einem nach innen gerichteten Umfangsflansch (19) versehen ist, wobei die Außenkanten der Klappen (9a-c) dichtend mit der nach innen gewandten Seitenfläche des Flansches (19) in Kontakt kommen.

8. Herzklappe nach einem der vorangehenden Ansprüche, wobei die Klappen (9a-c) in einer vollständig geöffneten Position einen Winkel ($\gamma$) gegenüber einer Linie parallel zu einer Mittellinie (L) des Rings (3) einnehmen, der größer ist als Null und kleiner als ungefähr 7°.

9. Herzklappe nach einem der vorangehenden Ansprüche, **dadurch gekennzeichnet**, daß sie aus einem Material besteht, das aus Titan und pyrolytischem Kohlenstoff ausgewählt wird.

## Revendications

1. Valve cardiaque comprenant une gaine ou bague qui est munie d'une ouverture centrale (4) et de volets qui peuvent être ouverts et fermés et qui ont pour objet de permettre l'écoulement du sang par l'ouverture centrale (4) dans un sens, où les volets (9a-c) sont conçus sous forme de segments de cercle, où l'angle de secteur ($\alpha$) a la valeur 360°/n, n étant le nombre de segment de cercle et étant égal à 3, 4, 5 ou 6, lesdits volets (9a-c) étant reliés de manière pivotante, au niveau de leur bord externe, à l'intérieur de la bague (3) à une extrémité de celle-ci, en ce que chaque volet (9a-c) est relié à la bague (3) au moyen d'une charnière qui est disposée au centre du bord externe du volet et qui est reçue dans un évidement dans ladite bague (3), de sorte que lesdits volets, dans une position fermée, coopèrent pour former un corps d'étanchéité et, dans une position ouverte, permettent un passage libre du sang, caractérisée en ce que les volets (9a-c) sont arqués de manière à former ensemble, dans la position fermée, un cône ayant un angle au sommet obtus ($\beta$) et en ce que chaque charnière contient une broche ou tige (15) qui est fixée à la bague (3) dans une ouverture (17) qui s'étend tangentiellement dans celle-ci.

2. Valve cardiaque selon la revendication 1, caractérisée en ce que le nombre de volets (9a-c) est de 3.

3. Valve cardiaque selon la revendication 1 ou 2, caractérisée en ce que ledit angle au sommet ($\beta$) est au plus de 180°.

4. Valve cardiaque selon la revendication 3, caractérisée en ce que ledit angle au sommet ($\beta$) est situé dans le domaine d'environ 150 à 175°.

5. Valve cardiaque selon l'une quelconque des revendications précédentes, caractérisée en ce que la fixation à la bague (3) est configurée de manière qu'un volet (9a-c) seul ne puisse pas être replié dans une position dépassant sensiblement la position qu'il occupe en coopération avec les autres volets (9a-c).

6. Valve cardiaque selon l'une quelconque des revendications précédentes, caractérisée en ce que la bague (3) est munie à ses extrémités de brides circonférentielles (5) formant un évidement (7) où une bague de suture peut être placée pour la fixation au tissu environnant.

7. Valve cardiaque selon l'une quelconque des revendications précédentes, caractérisée en ce que la bague (3) est munie à l'une de ses extrémités d'une bride circonférentielle (19) dirigée vers l'intérieur, les bords externes des volets (9a-c) entrant en contact de manière étanche avec la surface latérale de ladite bride (19) qui est tournée vers l'intérieur.

8. Valve cardiaque selon l'une quelconque des revendications précédentes, dans laquelle lesdits volets (9a-c) forment, dans une position totalement ouverte, un angle ($\gamma$) avec une droite parallèle à un axe (L) de ladite bague (3) qui est supérieur à 0 et inférieur à environ 7°.

9. Valve cardiaque selon l'une quelconque des revendications précédentes, caractérisée en ce qu'elle est constituée par un matériau choisi parmi le titane et le carbone pyrolytique.

*Fig.1*

*Fig. 2*

*Fig. 3*